Europäisches Patentamt

European Patent Office

Office européen des brevets

(11) Publication number: **0 311 107**
**A2**

(12) **EUROPEAN PATENT APPLICATION**

(21) Application number: **88116667.2**

(22) Date of filing: **07.10.88**

(51) Int. Cl.⁴: **C07H 19/173 , A61K 31/70**

(30) Priority: **08.10.87 US 107392**

(43) Date of publication of application:
**12.04.89 Bulletin 89/15**

(84) Designated Contracting States:
**AT BE CH DE ES FR GB GR IT LI LU NL SE**

(71) Applicant: **Stichting REGA V.Z.W.**
**Minderbroedersstraat 10**
**B-3000 Leuven(BE)**

(72) Inventor: **Balzarini, Jan Maria Réné**
**Prospor Poulletlaan 10/9**
**B-3000 Leuven(BE)**
Inventor: **Herdewijn, Piet**
**Diestsesteenweg 232**
**B-3200 Kessel-Lo(BE)**
Inventor: **De Clercq, Erik Désiré A.**
**Paul Lebrunstraat 35/7**
**B-3000 Leuven(BE)**

(74) Representative: **Prins, Hendrik Willem et al**
**Octrooibureau Arnold & Siedsma**
**Sweelinckplein, 1**
**NL-2517 GK The Hague(NL)**

(54) **Anti-HIV active 3'-fluoro-purine-2',3'-dideoxyribosides.**

(57) The novel 2′,3′-dideoxynucleosides of which the sugar moiety is fluorinated at the 3′ position: 3′-fluoro-2,6-diaminopurine 2′,3′-dideoxyriboside; and 3′-fluoro-2′,3′-dideoxyguanosine, selectively inhibit the cytopathogenicity and replication of retroviruses (i.e. human immunodeficiency virus).

FIG.2

## Anti-HIV active 3'-fluoro-purine-2',3'-dideoxyribosides

This invention relates to novel sugar-modified derivatives of purine-2',3'-dideoxyribosides, to their application as novel therapeutic agents as well as to their preparation.

A series of purine and pyrimidine nucleoside analogues with the sugar moiety in the 2',3'-dideoxyribose configuration has proven successful in the inhibition of the replication of human immunodeficiency virus (HIV) in vitro (Balzarini, J. et al, Biochem. Biophys. Res. Commun. 140:735-742 (1986a); Balzarini, J. et al, Mol. Pharmacol. 32:162-167 (1987a); Baba, M. et al, Biochem. Biophys. Res. Commun. 142:128-134 (1987a)). The most potent and selective inhibitors so far are 2',3'-dideoxycytidine (ddCyd), 2',3'-dideoxythymidine (ddThd), the 2',3'-unsaturated pyrimidine 2',3'-dideoxynucleoside analogues derived thereof (ddddCyd and ddddThd) (Balzarini, J. et al (1987a)), 5-fluoro-ddCyd (Kim et al, J. Med. Chem. 30:862-866 (1987)), 3'-fluoroddThd (FddThd) (Herdewijn, P. et al, J. Med. Chem. 30:12701278 (1987a)), 2',3'-dideoxyadenosine (ddAdo), 3'-azido-ddAdo (AzddAdo) (Herdewijn, P. et al, J. Med. Chem., (1987b)), 2',3'-dideoxyguanosine (ddGuo), and 2',3'-dideoxyinosine (ddIno). Recently, we reported on 2,6-diaminopurine 2',3'-dideoxyriboside (ddDAPR) (Balzarini, J. et al, Biochem. Biophys. Res. Commun. 145:269-276 (1987b)) as a potential new anti-AIDS drug. ddDAPR is, like ddAdo, a potent and selective inhibitor of HIV in vitro, and inhibits HIV-replication in MT4 cells at a 50% effective dose (ED$_{50}$) of 2.5-3.6 $\mu$M.

During further research for new potential anti-retroviral drugs we synthesized new 3' fluorinated purine-2',3'-dideoxyribosides.

We found that 3'-fluoro-2,6-diaminopurine 2',3'-dideoxyriboside (FddDAPR) and 3'-fluoro-guanine 2',3'-dideoxyriboside (3'-fluoro-2',3'-dideoxyguanosine) (FddGuo) are potent and selective anti-retrovirus agents in vitro. Their potency and selectivity as anti-HIV agents are close to ddDAPR.

Hereafter, FddDAPR and FddGuo will be compared with several other 2',3'-dideoxynucleosides.

## MATERIALS AND METHODS

2,6-Diamino-9-(2,3-dideoxy-$\beta$-D-glycero-pentofuranosyl)purine (2,6-diaminopurine 2',3'-dideoxyriboside, ddDAPR) was synthesized according to a procedure used for conversion of adenosine into its 2',3'-unsaturated derivative 2',3'-didehydro-2',3'-dideoxyadenosine (ddddAdo) and subsequent hydrogenation of ddddAdo to give ddAdo (McCarthy Jr., et al, J. Am. Chem. Soc. 88:1549-1553 (1966); Robins, M.J. et al, Tetrahedron Lett. 25:367-370 (1984); Hansske, F. et al, Tetrahedron Lett. 26:4295-4298 (1985)).

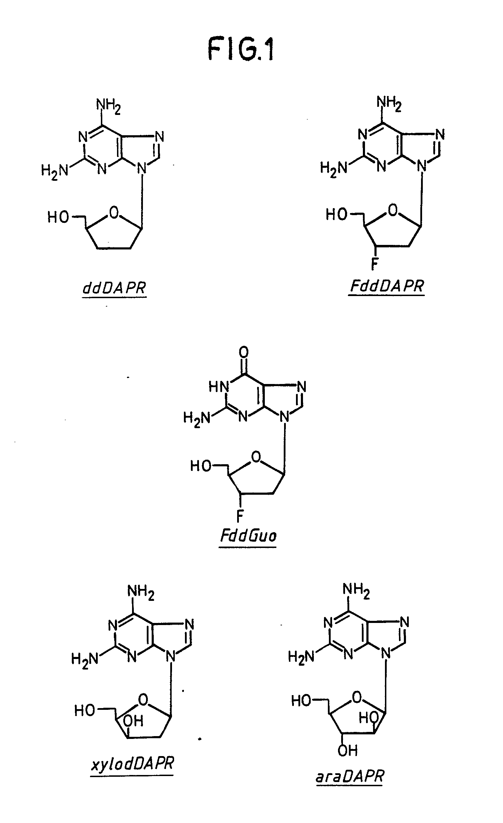

3'-Fluoro-ddDAPR (FddDAPR) was synthesized from 9-(5-O-trityl-2-deoxy-$\beta$-D-threo-pentofuranosyl)-2-N-trityl2,6-diaminopurine with diethylaminosulfurtrifluoride in dichloromethane, followed by deprotection with 80% acetic acid. The synthesis of 9-$\beta$-D-arabinofuranosyl 2,6-diaminopurine (araDAPR) has been described previously (Hansske F. et al, Tetrahedron, 40:125-135 (1984)). The structural formulae of ddDAPR, FddDAPR, FddGuo, XylodDAPR and araDAPR are depicted in Fig. 1. 2',3'-Dideoxyguanosine (ddGuo) was obtained from Pharmacia PL-Biochemicals (Uppsala. Sweden). 3'-Fluoro-2',3'-dideoxyguanosine (FddGuo) was obtained by deaminating FddDAPR with beef intestine adenosine deaminase (Boehringer Mannheim, Mannheim, West Germany). All reagents used were of the highest quality available.

Therapeutic compositions, containing FddDAPR and/or FddGuo respectively as an active ingredient for treating AIDS, AIDS-related diseases and other retroviral diseases including hepatitis B, in human practice may take the form of powders, suspensions, solutions, sprays, emulsions, unguents or creams and may be used for local application, for intranasal, rectal, vaginal and also for oral or parenteral (intravenous, intradermal, intramuscular, intrathecal etc.) administration. Such compositions may be prepared by combining (e.g. mixing, dissolving etc.) the active compound with pharmaceutically acceptable excipients of neutral, character (such as aqueous or non-aqueous solvents, stabilizers, emulsifiers, detergents, additives), and further, if necessary with dyes and aromatizers. The concentration of the active ingredient in the therapeutic composition may vary widely between 0.1% and 100%, dependent on the mode of administration. Further, the dose of the active ingredient to be administered may vary between 0.1 mg and 100 mg per kg of body weight.

The pharmaceutical properties of FddDAPR and FddGuo concerning their anti-retroviral action, especially HIV, are documented by the following examples which should not be read in a restrictive sense.

In the examples reference is made to the attached drawings in which:

Fig. 1 comprises structural formulae of ddDAPR, FddDAPR, FddGuo, XylOdDAPR and araDAPR,.

Fig. 2 is a bar representation of the test results on the inhibition of the cytopathogenic effect of HIV in human T4 lymphocyte MT4 cells by ddDAPR, FddDAPR, and FddGuo. The viability of the cells was measured by trypan blue exclusion after an incubation period of 5 days at 37° C.

## CELLS

The cultivation of Raji, Molt/4F, CEM, H9, HUT-78 and MT4 cells has been described in Balzarini, J, et al, Biochem. Biophys. Res. Commun. 136:64-71 (1986b) and Pauwels, R. et al, J. Virol. Methods, 16:171-185 (1987).

## VIRUSES

HIV was obtained from the culture supernatant of a H9 cell line persistently infected with HTLV-III$_B$. Moloney murine sarcoma virus (MSV) was prepared from tumors induced by in vivo infection of 2-3 day old NMRI mice.

## ANTIVIRAL ASSAYS

For transformation of C3H mouse embryo cells by Molo ney murine sarcoma virus (MSV), confluent monolayers of C3H cells in 1 ml wells of Tissue Culture Cluster plates were infected wiht 150 foci-forming units of MSV during 90 min, whereafter medium was replaced by 1 ml fresh culture medium containing different concentrations of test compound. After 6-7 days incubation at 37° C, transformation of the cell cultures was monitored microscopically.

For the determination of the cytopathic effect of HIV in human T-lymphocyte MT4 cells, MT4 cells, subcultured one day before the start of the experiment, were adjusted at $5 \times 10^5$ cells/ml and infected with HIV (HTLV-III$_B$) at 100 CCID$_{50}$/ml. Then, 100 $\mu$l of the infected cell suspension was brought into wells of a microtiter tray containing 100 $\mu$l of various dilutions of the test compounds. After 5 days incubation at 37° C, the number of viable cells was recorded microscopically in a hematocytometer by trypan blue exclusion.

The inhibitory effects of the test compounds on HIV antigen expression in infected H9 or HUT-78 cells were determined at day 8 and 14, respectively after infection, by indirect immunofluorescence and laser flow cytofluorography using a polyclonal antibody as probe, as previously described (Pauwels, R. et al, (1987)).

## ANTI-RETROVIRAL EFFECT

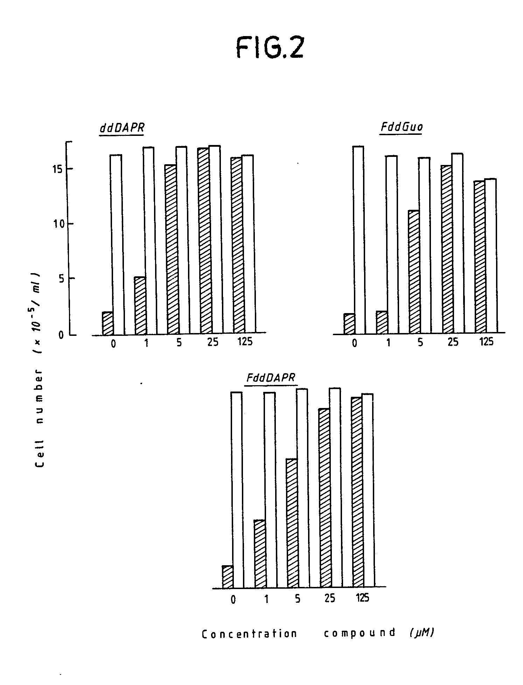

A series of novel 2,6-diaminopurine and guanine 2′,3′-dideoxynucleoside analogues in which the 2′,3′-dideoxyribose moiety is substituted by a 3′-fluoro group, or replaced by arabinose or 2′-deoxyxylose were evaluated for their effect on the transformation of C3H mouse embryo cells by Moloney murine sarcoma virus (MSV) (Table 1) and the cytopathogenicity induced by HIV in human T4 lymphocyte MT4 cells (Table 1, Fig. 2). FddDAPR selectively inhibited HIV-induced cytopathogenicity in MT4 cells. The anti-HIV activity of FddDAPR was comparable to that of FddGuo. The 9-β-D-arabinoside derivative and 2′-deoxy-9-β-D-xyloside derivative of ddDAPR are devoid of any anti-HIV activity.

To confirm our anti-HIV data obtained in the MT4 cell model, we also evaluated the 3′-fluorinated ddDAPR derivatives in two other human T4 lymphocyte cell lines (H9 and HUT-78) for their inhibitory effect on viral antigen expression (data not shown). In these experiments, FddDAPR achieves 50% inhibition of HIV-induced antigen expression at a concentration range of 0.05 - 1.0 $\mu$M. This concentration range is lower than that required to inhibit HIV-induced cytopathogenicity in MT4 cells.

## CYTOSTATIC AND ANTIMETABOLIC EFFECTS

The cytostatic effects of the compounds listed in Table 1 were examined against several human B-lymphoblast Raji, T-lymphoblast Molt/4F and T-lymphocyte CEM, H9 and MT4 cell lines (Table 2). None of the compounds evaluated showed severe cytostatic effects against any of the cell lines. In no case is the $ID_{50}$ of the compounds for tumor cell proliferation lower than 60 $\mu$M. FddGuo is slightly more cytostatic than FddDAPR ($ID_{50}$ : 190 to 228 $\mu$M, and 233 to >1000 $\mu$M, respectively). XylodDAPR and araDAPR are devoid of any anti-cellular activity, even when tested at a concentration as high as 1000 $\mu$M.

Cellular DNA synthesis as monitored by the incorporation of [methyl-$^3$H]dThd into DNA of MT4 cells is not significantly affected by the test compounds at 200 $\mu$M.

## EFFECT OF 2'-DEOXYCOFORMYCIN ON THE ANTI-RETROVIRAL AND CYTOSTATIC EFFECTS OF ddDAPR AND FddDAPR

The effect of preincubation of the cells by 10 $\mu$M 2'-deoxycoformycin (DCF) on the anti-HIV and anti-MSV activity, and the cytostatic action of ddDAPR and FddDAPR has been evaluated (Table 3). Pretreatment of MT4 or C3H cells with DCF resulted in a significantly decreased anti-retroviral effect of ddDAPR and FddDAPR. Also, pretreatment of Molt/4F, CEM and MT4 cells by DCF decreases considerably the cytostatic action of these compounds (table 4).

## SUBSTRATE ACTIVITIES OF THE ddDAPR ANALOGUES FOR BEEF INTESTINE ADENOSINE DEAMINASE

All four ddDAPR analogues were examined for their susceptibility to deamination by beef intestine adenosine de aminase (ADA). The $K_m$ values of ADA for the natural substrates adenosine (Ado) and 2'-deoxyadenosine (dAdo) are 29 $\mu$M and 15 $\mu$M, respectively (Table 5, Balzarini, J. et al, Biochem. Biophys. Res. Commun. 145:277-283 (1987c)). ddDAPR shows a similar $K_m$ than Ado but a $V_{max}$ that is only 3% of that observed for Ado. FddDAPR has a 4 to 5-fold higher $K_m$ (148 $\mu$M) and $V_{max}$ (31 $\mu$mole/mg protein/min) than ddDAPR Both araDAPR and XylodDAPR have similar $K_m$ and $V_{max}$ values, and their $V_{max}/K_m$ ratios are considerably lower than those recorded for ddDAPR and dAdo (table 5).

Because of their prominent anti-HIV and anti-MSV activity and their relatively non-toxic properties in cell culture, FddDAPR and FddGuo must be considered as two novel, potent and selective anti-retroviral agents, that should be further pursued for their efficacy in the treatment of retrovirus infections including AIDS and hepatitis B.

The close similarity of the biological properties of ddDAPR and FddDAPR to those of ddGuo and FddGuo and the fact that ddDAPR and FddDAPR are susceptible to deamination by beef intestine adenosine deaminase suggest that the 2,6-diaminopurine 2',3'-dideoxynucleosides may hypothetically exert their anti-retrovirus activity, at least in part, as prodrugs of ddGuo or FddGuo, respectively.

The in vitro selectivity index of FddGuo, as based on the ratio of the 50% cytotoxic dose to the 50% antivirally effective dose, is slightly higher than that of ddGuo and comparable to ddDAPR. Its anti-HIV activity is also higher than that found for its corresponding 2,6-diaminopurine derivative FddDAPR.

Table 1

| Anti-retroviral and cytotoxic activity of sugar-modified purine 2',3'-dideoxynucleoside analogues | | | | | | |
|---|---|---|---|---|---|---|
| Compound | HIV-induced cytopathogenicity in MT4 cells | | | MSV-induced C3H cell transformation | | |
| | $ED_{50}(\mu M)$ | $CD_{50}(\mu M)$ | S.I. | $ED_{50}(\mu M)$ | $CD_{50}(\mu M)$ | S.I. |
| ddDAPR | 3.8 | 404 | 106 | 19 | $\geq$ 200 | $\geq$ 11 |
| FddDAPR | 4.5 | 360 | 80 | 28 | > 400 | > 15 |
| XylodDAPR | > 125 | > 625 | - | > 400 | > 400 | - |
| araDAPR | > 125 | > 625 | - | > 400 | > 400 | - |
| ddGuo | 7.6 | 486 | 64 | 72 | > 400 | > 5.5 |
| FddGuo | 2.4 | 231 | 96 | 12 | > 400 | > 23 |

Table 2

| Cytostatic effect of sugar-modified purine 2',3'-dideoxynucleoside analogues against human B- and T-cell lines | | | | | |
|---|---|---|---|---|---|
| Compound | Inhibitory dose-50 ($\mu$M) | | | | |
| | Raji | Molt/4F | CEM | H9 | MT4 |
| ddDAPR | 324 | 244 | 125 | 466 | 644 |
| FddDAPR | 753 | 425 | 233 | 377 | 337 |
| XylodDAPR | > 1000 | > 1000 | > 1000 | > 1000 | > 1000 |
| araDAPR | > 1000 | > 1000 | > 1000 | > 1000 | > 1000 |
| ddGuo | 903 | 380 | 142 | 531 | 942 |
| FddGuo | 107 | 228 | 119 | 194 | 197 |

Table 3

| Effect of 2'-deoxycoformycin (DCF) on the anti-retrovirus activity of ddDAPR and FddDAPR | | | |
|---|---|---|---|
| Compound | Presence of 10 $\mu$M DCF | Effective dose-50 ($\mu$M) | |
| | | HIV-induced cytopathogenicity in MT4 cells | MSV-induced C3H cell transformation |
| ddDAPR | - | 3.8 | 23 |
| | + | 13 | 84 |
| FddDAPR | - | 4.5 | 31 |
| | + | 39 | 87 |

Table 4

| Effect of 2'-deoxycoformycin (DCF) on the cytostatic activity of ddDAPR and FddDAPR | | | | |
|---|---|---|---|---|
| Compound | Presence of DCF | Inhibitory dose-50 ($\mu$M) | | |
| | | CEM | Molt/4F | MT4 |
| ddDAPR | - | 128 | 220 | > 1000 |
| | + | > 1000 | > 1000 | > 1000 |
| FddDAPR | - | 241 | 353 | 469 |
| | + | 739 | 865 | 520 |

## EP 0 311 107 A2

Table 5

| Kinetic properties of beef intestine adenosine deaminase for 2,6-diaminopurine $2',3'$-dideoxynucleoside analogues | | | |
|---|---|---|---|
| Compound | $K_m$ ($\mu$M) | $V_{max}$ ($\mu$mole/mg proten/min) | $V_{max}/K_m$ |
| Ado | 29[a] | 287[a] | 9.9[a] |
| ddAdo | 73[a] | 262[a] | 3.6[a] |
| ddDAPR | 29[a] | 8.2[a] | 0.28[a] |
| araDAPR | 83 | 5.9 | 0.07 |
| xylodDAPR | 74 | 3.7 | 0.05 |
| FddDAPR | 148 | 31 | 0.21 |

[a] Data taken from Balzarini, J. et al (1987c)

**Claims**

1. $3'$-fluoro-$2',3'$-dideoxyguanosine.

2. $3'$-fluoro-2,6-diaminopurine-$2',3'$-dideoxyriboside.

3. A therapeutic composition for use in the treatment of retroviral diseases including hepatitis B, which comprises an active ingredient which is selected from the group consisting of $3'$-fluoro-purine-$2',3'$-dideoxyribosides.

4. A therapeutic composition as claimed in claim 3, wherein said $3'$-fluoro-purine-$2',3'$-dideoxyriboside is $3'$-fluoro-2,6-diaminopurine-dideoxyriboside.

5. A therapeutic composition as claimed in claim 3, wherein said $3'$-fluoro-purine-$2',3'$-dideoxyriboside is $3'$-fluoro-$2',3'$-dideoxyguanosine.

6. A therapeutic composition as claimed in claim 3, comprising said active ingredient in a concentration ranging from 0.1 - 100% by weight.

7. A therapeutic composition as claimed in claim 6, wherein said $3'$-fluoro-purine-$2',3'$-dideoxyriboside is $3'$-fluoro-2,6-diaminopurine-$2',3'$-dideoxyriboside.

8. A therapeutic composition as claimed in claim 6, wherein said $3'$-fluoro-purine-$2',3'$-dideoxyriboside is $3'$-fluoro-$2',3'$-dideoxyguanosine.

9. A therapeutic composition as claimed in claim 6, having the form which is selected from the group consisting of powders, suspensions, solutions, sprays, emulsions, unguents and creams.

10. A therapeutic composition for use in the treatment of AIDS or AIDS-related diseases which comprises an active ingredient, which is selected from the group consisting of $3'$-fluoropurine-$2',3'$-dideoxyribosides.

11. A therapeutic composition as claimed in claim 10, wherein said $3'$-fluoro-purine-$2',3'$-dideoxyriboside is $3'$-fluoro-2,6-diaminopurine-$2',3'$-dideoxyriboside.

12. A therapeutic composition as claimed in claim 10, wherein said $3'$-fluoro-purine-$2',3'$-dideoxyriboside is $3'$-fluoro-dideoxyguanosine.

13. A therapeutic composition as claimed in claim 7, comprising said active ingredient in a concentration ranging from 0.1 - 100% by weight.

14. A therapeutic composition as claimed in claim 8, having the form which is selected from the group consisting of powders, suspensions, solutions, sprays, emulsions, unguents and creams.

15. A method for the treatment of a retroviral disease which comprises administering of a drug which is selected from the group consisting of $3'$-fluoro-purine-$2',3'$-dideoxyribosides to a patient suffering from a retroviral disease, including hepatitis B.

16. A method as claimed in claim 15 wherein said $3'$-fluoro-purine-$2',3'$-dideoxyriboside is $3'$-fluoro-2,6-diaminopurine $2',3'$-dideoxyriboside.

17. A method as claimed in claim 15 wherein said $3'$-fluoro-purine-$2',3'$-dideoxyriboside is $3'$-fluoro-$2',3'$-dideoxyguanosine.

18. A method for the treatment of AIDS or AIDS-related diseases which comprises administering of a drug which is selected from the group consisting of 3'-fluoro-purine-2',3'-dideoxyribosides to a patient suffering from AIDS or AIDS-related diseases.

19. A method as claimed in claim 18, wherein said 3'-fluoro-purine-2',3'-dideoxyriboside is 3'-fluoro-2,6-diaminopurine 2',3'-dideoxyriboside.

20. A method as claimed in claim 18, wherein said 3'-fluoro-purine-2',3'-dideoxyriboside is 3'-fluoro-2',3'-dideoxyguanosine.

21. Use of an active ingredient selected from the group consisting of 3'-fluoro-purine-2',3'-dideoxyribosides for preparing a therapeutic composition against a retroviral disease, including hepatitis B.

22. Use of an active ingredient selected from the group consisting of 3'-fluoro-purine-2',3'-dideoxyribosides for preparing a therapeutic composition against AIDS or AIDS-related diseases.

# FIG.1

ddDAPR

FddDAPR

FddGuo

xylodDAPR

araDAPR

# FIG.2